# EUROPEAN PATENT APPLICATION

(11) **EP 1 950 205 A1**
(43) Date of publication of application: **30.07.2008**
(21) Application number: 06821926.0
(22) Date of filing: 18.10.2006
(51) Int. Cl.: C07D 261/14, A61K 9/16, A61K 9/20, A61K 9/28, A61K 31/5377, A61K 47/02, A61K 47/10, A61K 47/12, A61K 47/14, A61K 47/16, A61K 47/20, A61K 47/22, A61K 47/26, A61K 47/32, A61K 47/36, A61K 47/38, A61K 47/44, A61P 29/00, A61P 37/02

(54) **METHOD FOR STABILIZATION OF ISOXAZOLE COMPOUND**

(30) Priority: 19.10.2005 JP 2005304589
(71) Applicant: Dainippon Sumitomo Pharma Co., Ltd., Chuo-ku Osaka-shi Osaka 541-0045 (JP)
(72) Inventor: NISHII, Hiroyuki, Ibaraki-shi, Osaka 5670878 (JP); MATSUOKA, Makoto, Ibaraki-shi, Osaka 5670878 (JP)
(74) Representative: Duckworth, Timothy John
(86) International application number: PCT/JP2006/320732
(87) International publication number: WO 2007/046411

(57) **Abstract**

3-[(1S)-1-(2-fluorobiphenyl-4-yl)ethyl]-5-[[amino(morpholin-4-yl)methylene]amino]isoxazole is an isoxazole compound useful as a therapeutic agent for an autoimmune disease. The isoxazole compound can be stabilized by using an antioxidant such as a metabisulfite, bisulfite, sulfite or dibutylhydroxytoluene. A pharmaceutical composition for oral administration comprising the isoxazole compound and the antioxidant is an excellent pharmaceutical composition, since the isoxazole compound contained in the composition is stabilized.

## Description

### TECHNICAL FIELD

The present invention relates to a method for stabilizing an isoxazole compound and a pharmaceutical composition for oral administration of the isoxazole compound. More particularly, it relates to a stabilization method in which the isoxazole compound useful as a therapeutic or prophylactic agent for autoimmune diseases, inflammatory diseases and the like is stabilized with an antioxidant, and a pharmaceutical composition for oral administration which contains the isoxazole compound as an active ingredient and in which said active ingredient is in a stabilized state.

### BACKGROUND ART

An isoxazole compound represented by the following formula I: 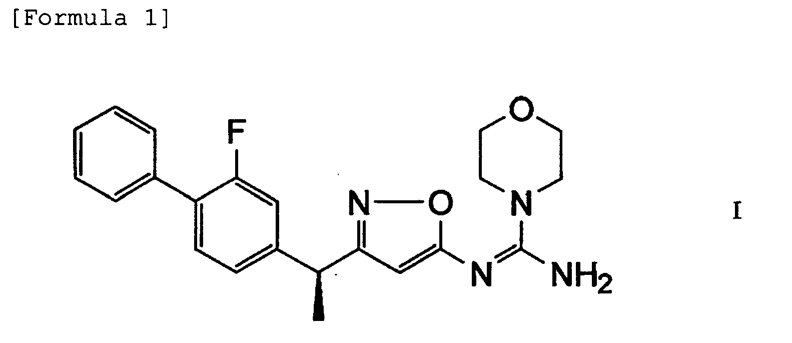 i.e., 3-[(1S)-1-(2-fluorobiphenyl-4-yl)ethyl]-5-{[amino(morpholin-4-yl)methylene]amino}isoxazole (in the present description, the term "the isoxazole compound" means this compound) is a compound useful as a therapeutic or prophylactic agent for autoimmune diseases (e.g. rheumatoid arthritis), inflammatory diseases and the like (patent document 1 and patent document 2).
Since the isoxazole compound is a difficultly water-soluble compound and has a low affinity for water, it is difficult to prepare this compound into a pharmaceutical composition for oral administration from which the compound is satisfactorily released by dissolution. Patent document 3 describes the fact that when a pulverized product of β type crystals of the isoxazole compound is made into a pharmaceutical composition for oral administration, this composition is obtainable as a pharmaceutical composition from which the compound is satisfactorily released by dissolution. Patent document 4 describes the preparation of pharmaceutical compositions for percutaneous administration (e.g. an ointment and a cream) of the isoxazole compound, and describes the fact that in the form of the pharmaceutical compositions for percutaneous administration, the isoxazole compound directly exhibits anti-inflammatory effect and anti-rheumatic effect in an affected part to which the composition is administered.

Patent document 1: International Patent Laid-Open No. WO98/47880 pamphlet
Patent document 2: JP-A-2000-186077
Patent document 3: International Patent Laid-Open No. WO02/092094 pamphlet
Patent document 4: International Patent Laid-Open No. WO03/068239 pamphlet

### DISCLOSURE OF THE INVENTION

### Problem to be Solved by the Invention

In general, when a drug is clinically used, the drug in a pharmaceutical composition is desired to be stable for a long period of time and moreover, stable even under severe conditions. The above-mentioned isoxazole compound is also desired to be in a stabilized state not only in a pharmaceutical composition but also as a starting drug before formulation. But, in particular, the stabilization has not yet been investigated in detail.
Therefore, an object of the present invention is to provide a method for stabilizing the isoxazole compound. Another object of the present invention is to provide a pharmaceutical composition for oral administration containing the isoxazole compound in a stabilized state.

### Means for Solving the Problem

The present inventors earnestly investigated the stabilization of the isoxazole compound in order to make it possible to produce industrially a pharmaceutical composition using the isoxazole compound, and consequently revealed the following: when a pharmaceutical composition of the isoxazole compound is stored in an ordinary state at room temperature, the isoxazole compound is decomposed to give a compound of the following formula II as a decomposition product: 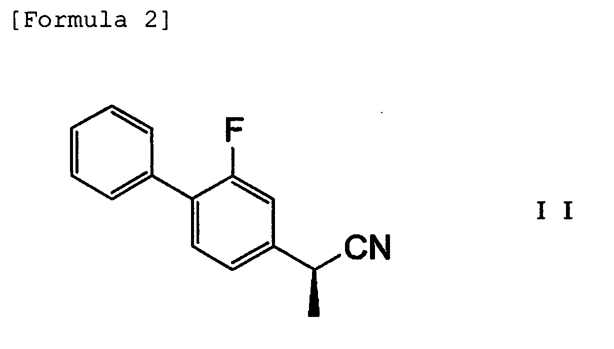 and in particular, (1) the amount of the compound produced by the decomposition of the isoxazole compound as starting drug before formulation tends to be increased with a decrease in the particle size of the isoxazole compound, and (2) the compression of the pharmaceutical composition into tablets increases the amount of the compound produced by the decomposition in the composition during storage after the compression. Accordingly, the present inventors considered that the desired stabilization of the isoxazole compound is achievable when the production of the compound by the decomposition is controllable. The present inventors further investigated and consequently found that unexpectedly, selective employment of a specific antioxidant permits satisfactory suppression of the production of the compound by the decomposition and hence permits the stabilization of the isoxazole compound, whereby the present invention has been accomplished.

Therefore, the present invention relates to a method for stabilizing the isoxazole compound represented by the following formula I: 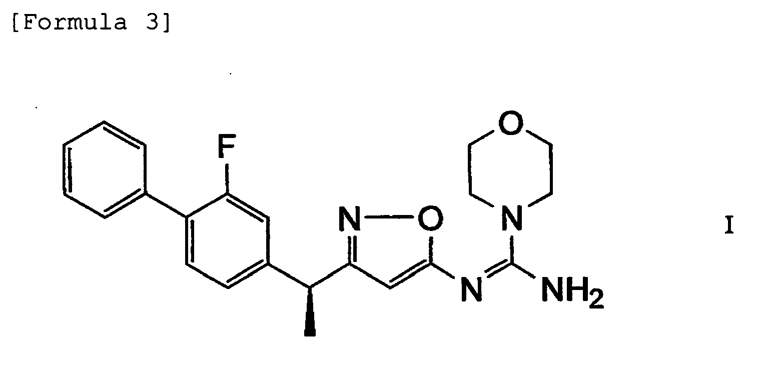 or a pharmaceutically acceptable salt thereof, characterized by stabilizing the isoxazole compound or the pharmaceutically acceptable salt thereof by suppressing the production of a compound of the following formula II: 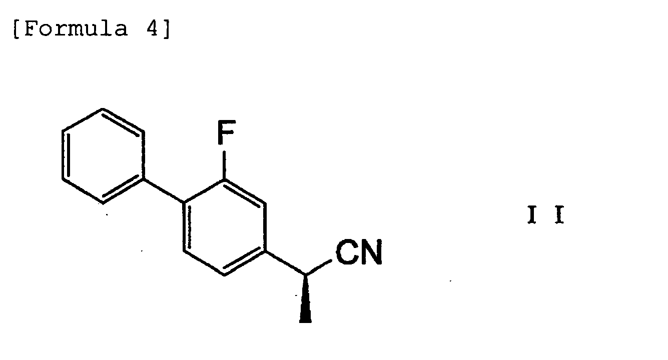 by the decomposition of the isoxazole compound or the pharmaceutically acceptable salt thereof, by the use of one or more antioxidants selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof.

In addition, the present invention relates to a pharmaceutical composition for oral administration comprising the isoxazole compound of the above formula I or a pharmaceutically acceptable salt thereof and one or more antioxidants selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof.

The present invention relates also to a pharmaceutical composition for oral administration comprising the following components (1) to (5) in the following proportions by weight:
(1) the isoxazole compound of the above formula I or a pharmaceutically acceptable salt thereof: 0.01% by weight to 25% by weight;
(2) a water-soluble excipient: 35% by weight to 90% by weight;
(3) a disintegrating agent: 1% by weight to 40% by weight;
(4) a water-soluble binder: 1% by weight to 10% by weight; and
(5) one or more antioxidants for suppressing the production of the compound of the above formula II by the decomposition which are selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof: at least 0.0008% by weight based on the weight of the isoxazole compound of the above formula I or the pharmaceutically acceptable salt thereof.

Further, the present invention relates to a pharmaceutical composition for oral administration which comprises the following components (1) to (5) in the following proportions by weight and is in such a stabilized state that when the composition is stored in a tight container at 25°C for 3 months, the amount of the compound of the above formula II produced by the decomposition is about 0.1% by weight or less based on the weight of the isoxazole compound of the formula I:
(1) the isoxazole compound of the above formula I or a pharmaceutically acceptable salt thereof: 0.01% by weight to 25% by weight;
(2) a water-soluble excipient: 35% by weight to 90% by weight;
(3) a disintegrating agent: 1% by weight to 40% by weight;
(4) a water-soluble binder: 1% by weight to 10% by weight; and
(5) at least one antioxidant selected from the group consisting of sodium metabisulfite, sodium bisulfite, sodium sulfite and dibutylhydroxytoluene: not more than 100% by weight and not less than 0.005% by weight based on the weight of the isoxazole compound of the above formula I or the pharmaceutically acceptable salt thereof.

Still further, the present invention relates to a pharmaceutical composition comprising the isoxazole compound of the above formula I or a pharmaceutically acceptable salt thereof in an amount of 90% by weight or more and at least one antioxidant selected from the group consisting of metabisulfites, bisulfites, sulfites and dibutylhydroxytoluene in an amount of 0.0008% by weight or more based on the weight of the isoxazole compound of the above formula I or the pharmaceutically acceptable salt thereof.

That is, specifically, the present invention is as described in the following items [1] to [40].
[1] A method for stabilizing the isoxazole compound represented by the following formula I: 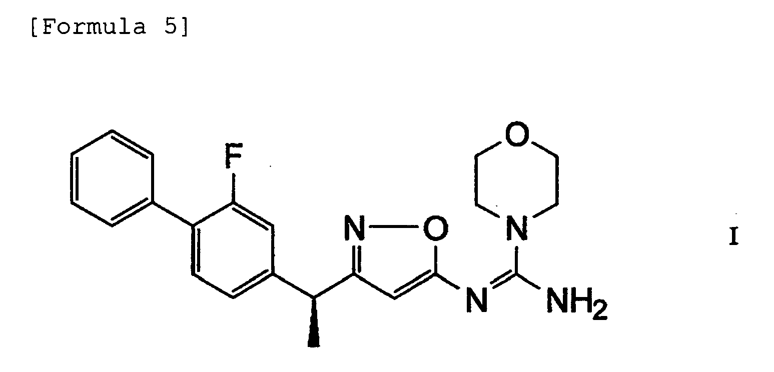 or a pharmaceutically acceptable salt thereof, characterized by stabilizing the isoxazole compound or the pharmaceutically acceptable salt thereof by suppressing the production of a compound of the following formula II: 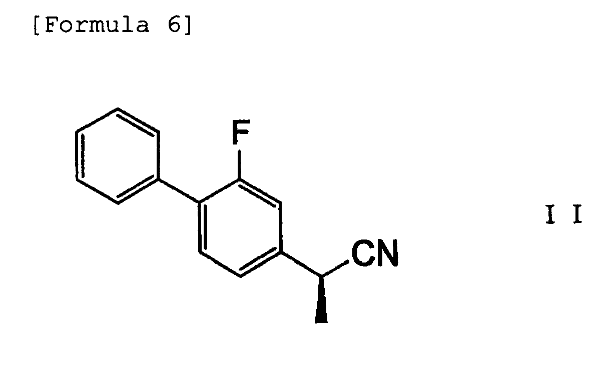 by the decomposition of the isoxazole compound or the pharmaceutically acceptable salt thereof, by the use of one or more antioxidants selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof.
[2] The stabilization method according to the above item [1], wherein the antioxidant(s) is at least one member selected from the group consisting of metabisulfites, bisulfites, sulfites, erythorbic acid or salts thereof, edetic acid or salts thereof, cysteine hydrochloride, dibutylhydroxytoluene, butylhydroxyanisole and propyl gallate.
[3] The stabilization method according to the above item [1] or [2], wherein the antioxidant(s) is at least one member selected from the group consisting of metabisulfites, bisulfites, sulfites and dibutylhydroxytoluene.
[4] The stabilization method according to any one of the above items [1] to [3], wherein the antioxidant(s) is at least one member selected from the group consisting of sodium metabisulfite, sodium bisulfite, sodium sulfite and dibutylhydroxytoluene.
[5] The stabilization method according to any one of the above items [1] to [4], wherein the antioxidant(s) is used in an amount of at least 0.0008% by weight based on the weight of the isoxazole compound or the pharmaceutically acceptable salt thereof.
[6] The stabilization method according to any one of the above items [1] to [5], wherein the antioxidant(s) is used in an amount of 100% by weight or less based on the weight of the isoxazole compound or the pharmaceutically acceptable salt thereof.
[7] The stabilization method according to any one of the above items [1] to [6], wherein the isoxazole compound of the above formula I is a pulverized product of β type crystals.
[8] A pharmaceutical composition for oral administration comprising the isoxazole compound of the above formula I or a pharmaceutically acceptable salt thereof and one or more antioxidants selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof.
[9] The pharmaceutical composition for oral administration according to the above item [8], wherein the antioxidant(s) is that intended for suppressing the production of the compound of the above formula II by the decomposition.
[10] The pharmaceutical composition for oral administration according to the above item [8] or [9], wherein the antioxidant(s) is at least one member selected from the group consisting of metabisulfites, bisulfites, sulfites, erythorbic acid or salts thereof, edetic acid or salts thereof, cysteine hydrochloride, dibutylhydroxytoluene, butylhydroxyanisole and propyl gallate.
[11] The pharmaceutical composition for oral administration according to any one of the above items [8] to [10], wherein the antioxidant(s) is at least one member selected from the group consisting of metabisulfites, bisulfites, sulfites and dibutylhydroxytoluene.
[12] The pharmaceutical composition for oral administration according to any one of the above items [8] to [11], wherein the antioxidant(s) is at least one member selected from the group consisting of sodium metabisulfite, sodium bisulfite, sodium sulfite and dibutylhydroxytoluene.
[13] The pharmaceutical composition for oral administration according to any one of the above items [8] to [12], which comprises the antioxidant(s) in an amount of at least 0.0008% by weight based on the weight of the isoxazole compound or the pharmaceutically acceptable salt thereof.
[14] The pharmaceutical composition for oral administration according to any one of the above items [8] to [13], which comprises the antioxidant(s) in an amount of at least 0.005% by weight based on the weight of the isoxazole compound or the pharmaceutically acceptable salt thereof.
[15] The pharmaceutical composition for oral administration according to any one of the above items [8] to [14], which comprises the antioxidant(s) in an amount of at least 0.025% by weight based on the weight of the isoxazole compound or the pharmaceutically acceptable salt thereof.
[16] The pharmaceutical composition for oral administration according to any one of the above items [8] to [15], which comprises the antioxidant(s) in an amount of at least 0.05% by weight based on the weight of the isoxazole compound or the pharmaceutically acceptable salt thereof.
[17] The pharmaceutical composition for oral administration according to any one of the above items [8] to [16], which comprises the antioxidant(s) in an amount of at least 0.25% by weight based on the weight of the isoxazole compound or the pharmaceutically acceptable salt thereof.
[18] The pharmaceutical composition for oral administration according to any one of the above items [8] to [17], which comprises the antioxidant(s) in an amount of 100% by weight or less based on the weight of the isoxazole compound or the pharmaceutically acceptable salt thereof.
[19] The pharmaceutical composition for oral administration according to any one of the above items [8] to [18], which is in the form of tablets or granules.
[20] The pharmaceutical composition for oral administration according to the above item [19], wherein the tablets are film-coated tablets.
[21] A pharmaceutical composition for oral administration comprising the following components (1) to (5) in the following proportions by weight:
   (1) the isoxazole compound of the above formula I or a pharmaceutically acceptable salt thereof: 0.01% by weight to 25% by weight;
   (2) a water-soluble excipient: 35% by weight to 90% by weight;
   (3) a disintegrating agent: 1% by weight to 40% by weight;
   (4) a water-soluble binder: 1% by weight to 10% by weight; and
   (5) one or more antioxidants for suppressing the production of the compound of the above formula II by the decomposition which are selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof: at least 0.0008% by weight based on the weight of the isoxazole compound of the above formula I or the pharmaceutically acceptable salt thereof.
[22] The pharmaceutical composition for oral administration according to the above item [21], which comprises the antioxidant(s) in an amount of at least 0.005% by weight based on the weight of the isoxazole compound of the above formula I or the pharmaceutically acceptable salt thereof.
[23] The pharmaceutical composition for oral administration according to the above item [21] or [22], wherein the antioxidant(s) is at least one member selected from the group consisting of metabisulfites, bisulfites, sulfites, erythorbic acid or salts thereof, edetic acid or salts thereof, cysteine hydrochloride, dibutylhydroxytoluene, butylhydroxyanisole and propyl gallate.
[24] The pharmaceutical composition for oral administration according to any one of the above items [21] to [23], wherein the antioxidant(s) is at least one member selected from the group consisting of metabisulfites, bisulfites, sulfites and dibutylhydroxytoluene.
[25] The pharmaceutical composition for oral administration according to any one of the above items [21] to [24], wherein the antioxidant(s) is at least one member selected from the group consisting of sodium metabisulfite, sodium bisulfite, sodium sulfite and dibutylhydroxytoluene.
[26] The pharmaceutical composition for oral administration according to any one of the above items [21] to [25], wherein the content of the isoxazole compound or the pharmaceutically acceptable salt thereof is not more than 25% by weight and not less than 0.1% by weight.
[27] The pharmaceutical composition for oral administration according to any one of the above items [21] to [26], wherein the content of the isoxazole compound or the pharmaceutically acceptable salt thereof is not more than 25% by weight and not less than 1% by weight.
[28] The pharmaceutical composition for oral administration according to any one of the above items [21] to [27], wherein the content of the antioxidant(s) is not more than 100% by weight and not less than 0.025% by weight based on the weight of the isoxazole compound or the pharmaceutically acceptable salt thereof.
[29] The pharmaceutical composition for oral administration according to any one of the above items [21] to [28], wherein the content of the antioxidant(s) is not more than 100% by weight and not less than 0.05% by weight based on the weight of the isoxazole compound or the pharmaceutically acceptable salt thereof.
[30] The pharmaceutical composition for oral administration according to any one of the above items [21] to [29], wherein the content of the antioxidant(s) is not more than 10% by weight and not less than 0.25% by weight based on the weight of the isoxazole compound or the pharmaceutically acceptable salt thereof.
[31] The pharmaceutical composition for oral administration according to any one of the above items [21] to [30], wherein the content of the water-soluble binder is not more than 5% by weight and not less than 1% by weight.
[32] The pharmaceutical composition for oral administration according to any one of the above items [21] to [31], the dosage form of which is tablets or granules.
[33] The pharmaceutical composition for oral administration according to any one of the above items [21] to [32], wherein the isoxazole compound of the above formula I is a pulverized product of β type crystals.
[34] The pharmaceutical composition for oral administration according to any one of the above items [21] to [33], wherein the water-soluble excipient is at least one member selected from the group consisting of lactose, mannitol, erythritol and xylitol.
[35] The pharmaceutical composition for oral administration according to any one of the above items [21] to [34], wherein the disintegrating agent is at least one member selected from the group consisting of croscalmellose sodium, sodium starch glycolate, crospovidone, carmellose calcium, low-substituted hydroxypropyl cellulose, partly pregelatinized starch and natural starch.
[36] The pharmaceutical composition for oral administration according to any one of the above items [21] to [35], wherein the water-soluble binder is at least one member selected from the group consisting of hydroxypropylmethyl cellulose, hydroxypropyl cellulose, polyvinylpyrrolidones, polyvinyl alcohols and pullulan.
[37] The pharmaceutical composition for oral administration according to any one of the above items [21] to [36], which is in such a stabilized state that when the composition is stored in a tight container at 25°C for 3 months, the amount of the compound of the above formula II produced by the decomposition is about 0.1% by weight or less based on the weight of the isoxazole compound of the formula I.
[38] A pharmaceutical composition for oral administration which comprises the following components (1) to (5) in the following proportions by weight and is in such a stabilized state that when the composition is stored in a tight container at 25°C for 3 months, the amount of the compound of the above formula II produced by the decomposition is about 0.1% by weight or less based on the weight of the isoxazole compound of the formula I:
   (1) the isoxazole compound of the above formula I or a pharmaceutically acceptable salt thereof: 0.01% by weight to 25% by weight;
   (2) a water-soluble excipient: 35 to 90% by weight;
   (3) a disintegrating agent: 1 to 40% by weight;
   (4) a water-soluble binder: 1 to 10% by weight; and
   (5) at least one antioxidant selected from the group consisting of sodium metabisulfite, sodium bisulfite, sodium sulfite and dibutylhydroxytoluene: not more than 100% by weight and not less than 0.005% by weight based on the weight of the isoxazole compound of the above formula I or the pharmaceutically acceptable salt thereof.
[39] The pharmaceutical composition for oral administration according to the above item [38], which comprises the antioxidant(s) in a proportion by weight of not more than 50% by weight and not less than 0.025% by weight based on the weight of the isoxazole compound of the above formula I or the pharmaceutically acceptable salt thereof.
[40] A pharmaceutical composition comprising the isoxazole compound of the above formula I or a pharmaceutically acceptable salt thereof in an amount of 90% by weight or more and at least one antioxidant selected from the group consisting of metabisulfites, bisulfites, sulfites and dibutylhydroxytoluene in an amount of 0.0008% by weight or more based on the weight of the isoxazole compound of the above formula I or the pharmaceutically acceptable salt thereof.

### Advantages of the Invention

When a specific antioxidant(s) excluding nitrites such as sodium nitrite and potassium nitrite is used in combination with the isoxazole compound, the production of the compound of the formula II by the decomposition is suppressed, so that long-term stabilization of the isoxazole compound is achieved and that the stabilization of the isoxazole compound is achieved even under severe conditions. Therefore, a pharmaceutical composition for oral administration (for example, that in the form of granules, tablets or film-coated tablets) containing the specific antioxidant(s) together with the isoxazole compound is an excellent composition containing the isoxazole compound in a stabilized state.

### BEST MODE FOR CARRYING OUT THE INVENTION

The isoxazole compound of the formula I is useful as a therapeutic agent for autoimmune diseases (e.g. rheumatoid arthritis), inflammatory diseases and the like, and may be produced by the production process described in U.S. Patent 6100260, International Patent Laid-Open No. WO98/47880 pamphlet, or the like. As the pharmaceutically acceptable salt of the isoxazole compound, acid addition salts are exemplified. The acid addition salts include, for example, inorganic acid addition salts such as hydrochloride, hydrobromide, sulfate, phosphate, nitrate, etc.; and organic acid addition salts such as acetate, propionate, succinate, lactate, malate, tartarate, citrate, maleate, fumarate, methanesulfonate, p-toluenesulfonate, ascorbate, etc. The isoxazole compound or the pharmaceutically acceptable salt thereof may be in the form of a hydrate or a solvate such as an alcohol solvate (e.g. ethanol solvate).
In the present invention, the isoxazole compound is preferably β type crystals of free form of the isoxazole compound, i.e., crystalline free form showing main peaks at angles of diffraction (2θ) of 6.1°, 14.1°, 16.0°, 18.5°, 20.0° and 25.4° in powder X-ray diffraction. The isoxazole compound is more preferably a pulverized product of such crystals having a D50% particle size (a median diameter calculated on a volume basis) of 15 µm or less, more preferably 10 µm or less, still more preferably 7 µm or less, in particular, 1 µm to 7 µm. Such β type crystals and a pulverized product thereof may be produced by the process described in International Patent Laid-Open No. WO02/092094 pamphlet.

The specific antioxidant(s) excluding nitrites such as sodium nitrite and potassium nitrite and used in the present invention is one or more antioxidants selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof. Specific examples of the antioxidant(s) used in the present invention are metabisulfites such as sodium metabisulfite, potassium metabisulfite, etc.; bisulfites such as sodium bisulfite, potassium bisulfite, etc.; sulfites such as sodium sulfite, potassium sulfite, etc.; erythorbic acid or erythorbic acid salts such as sodium erythorbate, potassium erythorbate, etc.; edetic acid (EDTA) or edetic acid salts such as sodium edetate, potassium edetate, calcium edetate disodium, etc.; cysteine hydrochloride; dibutylhydroxytoluene (BHT); butylhydroxyanisole (BHA); propyl gallate; ascorbic acid or ascorbic acid salts such as sodium L-ascorbate, calcium ascorbate, etc.; ascorbic acid esters such as L-ascorbic acid stearic acid esters, ascorbic acid palmitic acid ester, etc.; alpha-thioglycerol; citric acid or citric acid salts such as sodium citrate, calcium citrate, etc.; tocopherols such as natural vitamin E, dl-α-tocopherol, d-δ-tocopherol, etc.; tocopherol esters such as dl-α-tocopherol acetate, dl-α-tocopherol succinate, calcium dl-α-tocopherol succinate, etc.; lecithin; thioglycolic acid or thioglycolic acid salts such as sodium thioglycolate, calcium thioglycolate, etc.; and thiomalic acid or thiomalic acid salts such as sodium thiomalate, etc. Preferable examples of the antioxidant(s) are metabisulfites such as sodium metabisulfite, potassium metabisulfite, etc.; bisulfite such as sodium bisulfite, potassium bisulfite, etc.; sulfites such as sodium sulfite, potassium sulfite, etc.; erythorbic acid or erythorbic acid salts such as sodium erythorbate, potassium erythorbate, etc.; edetic acid or edetic acid salts such as sodium edetate, potassium edetate, etc.; cysteine hydrochloride; dibutylhydroxytoluene; butylhydroxyanisole; and propyl gallate. Of these, the metabisulfites such as sodium metabisulfite, potassium metabisulfite, etc.; the bisulfite such as sodium bisulfite, potassium bisulfite, etc.; the sulfites such as sodium sulfite, potassium sulfite, etc.; and dibutylhydroxytoluene are especially preferable. In particular, sodium metabisulfite, sodium bisulfite, sodium sulfite and dibutylhydroxytoluene are the most preferable. The antioxidant(s) used in the present invention includes hydrates of the above-exemplified antioxidants. The above-exemplified antioxidants may be used in combination.

The amount of the antioxidant(s) used in the present invention may be any amount as long as it is pharmacologically acceptable as the amount of each antioxidant. The amount is preferably at least 0.0008% by weight, more preferably at least 0.005% by weight, still more preferably at least 0.025% by weight, especially preferably at least 0.05% by weight, in particular, at least 0.25% by weight, based on the weight of the isoxazole compound or the pharmaceutically acceptable salt thereof. The upper limit of the amount of the antioxidant(s) used in the present invention is any amount as long as it is pharmaceutically acceptable. The upper limit is preferably 100% by weight or less, more preferably 65% by weight or less, still more preferably 50% by weight or less, especially preferably 20% by weight or less, in particular, 10% by weight or less, most preferably 2.5% by weight or less, based on the weight of the isoxazole compound or the pharmaceutically acceptable salt thereof.

In the present invention, to stabilize the isoxazole compound or a pharmaceutically acceptable salt thereof by suppressing the production of the compound of the formula II by the decomposition, by the use of the antioxidant(s), it is sufficient that the isoxazole compound or the pharmaceutically acceptable salt thereof and the antioxidant(s) are made present together. A method for making them present together is not particularly limited. The stabilization may be achieved, for example, by spraying the isoxazole compound or pharmaceutically acceptable salt thereof as a starting drug with an aqueous solution obtained by dissolving the antioxidant(s) in purified water in an amount corresponding to the above-mentioned preferable amount, and then drying the compound or salt to obtain a dried product. Even when such a dried product is stored for a long period of time in a usually employed environment or under severe conditions, it is possible to keep the isoxazole compound or the pharmaceutically acceptable salt thereof in a stabilized state. When the antioxidant(s) used is a water-insoluble antioxidant(s) such as dibutylhydroxytoluene or butylhydroxyanisole, the antioxidant(s) may be used after being dissolved in a mixture of water and an alcohol such as ethanol. As the mixture, a mixture of water and an alcohol is preferable. Although the mixing ratio is not limited, a mixture of water and an alcohol in a weight ratio of 1 : 1 may be used.

The stabilization of the isoxazole compound or the pharmaceutically acceptable salt thereof may be achieved also when the isoxazole compound or the pharmaceutically acceptable salt thereof is incorporated as an active ingredient into a pharmaceutical composition of a conventional type and the above-mentioned specific antioxidant(s) is made present together with the compound or the salt by a conventional method to prepare a desired pharmaceutical composition. Even when such a pharmaceutical composition is stored for a long period of time in a usually employed environment or under severe conditions, the isoxazole compound or the pharmaceutically acceptable salt thereof is kept stable. As such a pharmaceutical composition, the pharmaceutical composition for oral administration hereinafter explained in detail is preferable. The pharmaceutical composition for oral administration is preferably in a form suitable for oral administration, such as tablets, granules, powder, pills, capsules or the like. Such a pharmaceutical composition can easily be prepared by a conventional method by the use of an excipient, a binder, a disintegrating agent and the like, which are usually used.

In the present invention, as is clear from the above, a pharmaceutical composition for oral administration comprising the isoxazole compound or a pharmaceutically acceptable salt thereof and the antioxidant(s) is provided as an excellent pharmaceutical composition in which the isoxazole compound or the pharmaceutically acceptable salt thereof is in a stabilized state. The antioxidant(s) used here suppresses the production of the compound of the formula II by the decomposition as described above. As the antioxidant(s) used here, the above-exemplified specific antioxidant(s) is used in the same manner as above and the amount of the antioxidant(s) used here is also the same as above.
Such a pharmaceutical composition for oral administration is preferably in the form of tablets or granules, in particular, film-coated tablets. When the composition is in such a form, it is preferably a pharmaceutical composition for oral administration comprising the following components (1) to (5) in the following proportions by weight:
(1) the isoxazole compound or a pharmaceutically acceptable salt thereof: 0.01% by weight to 25% by weight;
(2) a water-soluble excipient: 35% by weight to 90% by weight;
(3) a disintegrating agent: 1% by weight to 40% by weight;
(4) a water-soluble binder: 1% by weight to 10% by weight; and
(5) one or more antioxidants for suppressing the production of the compound of the formula II by the decomposition which are selected from metabisulfites; bisulfites; sulfites; erythorbic acid or salts thereof; edetic acid (EDTA) or salts thereof; cysteine hydrochloride; dibutylhydroxytoluene (BHT); butylhydroxyanisole (BHA); propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof: at least 0.0008% by weight based on the weight of the isoxazole compound or the pharmaceutically acceptable salt thereof.

The isoxazole compound used here is preferably the above-mentioned pulverized product of β type crystals. Specifically, the antioxidant(s) is preferably the same as that described above. The water-soluble excipient includes, for example, sugars such as lactose, sucrose, fructooligosaccharide, palatinose, glucose, maltose, reducing maltose, maltose syrup powder, fructose, isomerized lactose, reducing lactose, honey sugar, etc.; and sugar alcohols such as mannitol, erythritol, xylitol, sorbitol, maltitol, etc. Of these, lactose, mannitol, erythritol, xylitol and the like are preferable. The disintegrating agent includes, for example, croscalmellose sodium, sodium starch glycolate, crospovidone, carmellose calcium, low-substituted hydroxypropyl cellulose, partly pregelatinized starch, natural starches (e.g. corn starch and potato starch), crystalline cellulose, carmellose, carmellose sodium, anhydrous calcium hydrogenphosphate, calcium phosphate, magnesium aluminate metasilicate, synthetic hydrotalcite, and synthetic aluminum silicate. Of these, croscalmellose sodium, sodium starch glycolate, crospovidone, carmellose calcium, low-substituted hydroxypropyl cellulose, natural starches (e.g. corn starch and potato starch) and the like are preferable. The water-soluble binder includes, for example, hydroxypropylmetyl cellulose (hypromellose), hydroxypropyl cellulose, polyvinylpyrrolidones, polyvinyl alcohols, pullulan, starch, dextrin and gelatin. Of these, hydroxypropylmetyl cellulose, hydroxypropyl cellulose, polyvinylpyrrolidones, polyvinyl alcohols, pullulan and the like are preferable. The above-exemplified water-soluble excipients, disintegrating agents or water-soluble binders may be used in combination, respectively.

The above-mentioned pharmaceutical composition for oral administration preferably contains the antioxidant(s) in an amount of at least 0.005% by weight, more preferably not more than 100% by weight and not less than 0.025% by weight, in particular, not more than 100% by weight and not less than 0.05% by weight, still more preferably not more than 10% by weight and not less than 0.25% by weight, based on the weight of the isoxazole compound of the above formula I or the pharmaceutically acceptable salt thereof. The content of the isoxazole compound or the pharmaceutically acceptable salt thereof in the pharmaceutical composition for oral administration is preferably not more than 25% by weight and not less than 0.1% by weight, in particular, not more than 25% by weight and not less than 1% by weight. The content of the water-soluble binder is preferably not more than 5% by weight and not less than 1% by weight.

The above-mentioned pharmaceutical composition for oral administration is preferably in such a stabilized state that when the composition is stored in a tight container at 25°C for 3 months, the amount of the compound of the above formula II produced by the decomposition is about 0.1% by weight or less based on the weight of the isoxazole compound of the formula I.
In the present invention, preferable is a pharmaceutical composition for oral administration which comprises the following components (1) to (5) in the following proportions by weight and is in such a stabilized state that when the composition is stored in a tight container at 25°C for 3 months, the amount of the compound of the above formula II produced by the decomposition is about 0.1% by weight or less based on the weight of the isoxazole compound of the formula I:
(1) the isoxazole compound of the above formula I or a pharmaceutically acceptable salt thereof: 0.01% by weight to 25% by weight;
(2) a water-soluble excipient: 35 to 90% by weight;
(3) a disintegrating agent: 1 to 40% by weight;
(4) a water-soluble binder: 1 to 10% by weight; and
(5) at least one antioxidant selected from the group consisting of sodium metabisulfite, sodium bisulfite, sodium sulfite and dibutylhydroxytoluene: not more than 100% by weight and not less than 0.005% by weight based on the weight of the isoxazole compound of the above formula I or the pharmaceutically acceptable salt thereof.
   This pharmaceutical composition for oral administration preferably contains the antioxidant(s) in a proportion by weight of not more than 50% by weight and not less than 0.025% by weight based on the weight of the isoxazole compound of the above formula I or the pharmaceutically acceptable salt thereof.
   In the present invention, preferable is a pharmaceutical composition comprising the isoxazole compound of the above formula I or a pharmaceutically acceptable salt thereof in an amount of 90% by weight or more and at least one antioxidant selected from the group consisting of metabisulfites, bisulfites, sulfites and dibutylhydroxytoluene in an amount of 0.0008% by weight or more based on the weight of the isoxazole compound of the above formula I or the pharmaceutically acceptable salt thereof.

The following process is preferable for producing the pharmaceutical composition for oral administration in the form of tablets, film-coated tablets, granules or the like by the use of the above-mentioned components (1) to (5).
The tablets or the film-coated tablets are produced, for example, as follows. The above-mentioned water-soluble excipient and disintegrating agent are charged into a conventional fluidized-bed granulator and sprayed with a binding liquid obtained by dispersing and suspending the isoxazole compound or a pharmaceutically acceptable salt thereof in an aqueous solution of the water-soluble binder, and then with an aqueous solution of the antioxidant(s) to obtain a granulated product, and a conventional lubricant (e.g. magnesium stearate) and the like are blended with the granulated product, followed by compression with a conventional tabletting machine, whereby the tablets may be produced. In such a production process, the following is also possible: the isoxazole compound or a pharmaceutically acceptable salt thereof and the antioxidant(s) are added to an aqueous solution of the above-mentioned water-soluble binder and dispersed and suspended therein to prepare a binding liquid, and the binding liquid is sprayed on the water-soluble excipient and disintegrating agent charged into a fluidized-bed granulator to obtain a granulated product. It is also possible to charge the isoxazole compound or a pharmaceutically acceptable salt thereof into a fluidized-bed granulator together with the water-soluble excipient and the disintegrating agent and spray them with an aqueous solution of the water-soluble binder and the antioxidant(s) as a binding liquid.
In the above-mentioned production process, when the antioxidant(s) is a water-insoluble antioxidant(s) such as dibutylhydroxytoluene or butylhydroxyanisole, a mixture of water and an alcohol (e.g. ethanol) (although the mixing ratio is not limited, for example, a mixture of water and an alcohol in a weight ratio of 1 : 1 may be used) may be used, and the binding liquid may be prepared by the use of such a mixture.

By coating the tablets obtained in the manner described above with film if necessary, film-coated tablets may be produced. As a coating agent, there are exemplified combinations of a base material such as hydroxypropylmethyl cellulose, hydroxypropyl cellulose, polyvinylpyrrolidone or the like and a plasticizer such as polyethylene glycol, propylene glycol, triacetin, triethyl citrate, glycerol, glycerol fatty acid ester, polyethylene glycol or the like. Additives such as titanium oxide, mannitol, etc. may be added according to need. It is also possible to prepare a film-coating liquid by the use of a premix of the above-mentioned coating agent. In this case, the premix is available as Opadry, a trade name, from Colorcon Japan. As a coating apparatus, a coating pan is exemplified.

The granules may be obtained, for example, by mixing the isoxazole compound or a pharmaceutically acceptable salt thereof with the above-mentioned water-soluble excipient and disintegrating agent to obtain a mixture, subjecting the mixture to wet granulation by using an aqueous solution of the above-mentioned water-soluble binder and antioxidant(s) as a binding liquid, and then drying the granulated product, followed by particle size regulation. If necessary, a fluidizing agent and the like may be incorporated into the granulated product. In this case, when the antioxidant(s) is a water-insoluble antioxidant(s) such as dibutylhydroxytoluene or butylhydroxyanisole, the binding liquid may be prepared by the use of a mixture of water and an alcohol such as ethanol as in the above-mentioned production of the tablets.
Thus, the pharmaceutical composition for oral administration of the present invention in the form of tablets, film-coated tablets, granules or the like is produced. In such a pharmaceutical composition, the isoxazole compound or a pharmaceutically acceptable salt thereof is in a satisfactorily stabilized state as an active ingredient.

The present invention is illustrated in further detail with the following working examples, reference examples and test examples, which should not be construed as limiting the scope of the invention.

### Reference Example 1

### Confirmation and identification of a compound produced by the decomposition of the isoxazole compound

The isoxazole compound was stored under oxidizing conditions (dissolution in an acetonitrile/water (1/1) mixture containing 1% hydrogen peroxide, followed by storage at 40°C for 3 days) or severe conditions (storage in a solid state at 130°C for 6 hours), and a compound produced as a main decomposition product by the storage was separated and then identified as a compound of the following formula II: 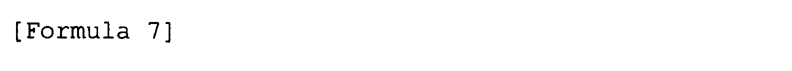 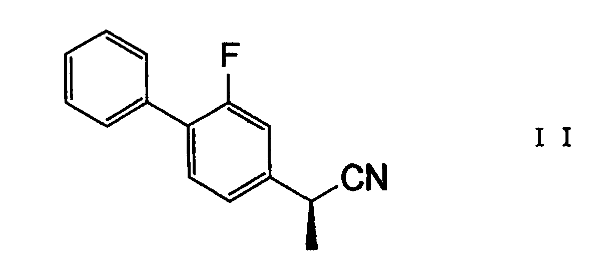 ¹H NMR(300MHz,CDCl₃)δ 1.68(d,3H,J=7.1Hz), 3.94(q,1H,J=7.1Hz),7.15-7.26(m,2H),7.36-7.55(m,6H)
The compound of the formula II produced by the decomposition is hereinafter abbreviated as the decomposition product (II).
Since it had been confirmed that the main decomposition product produced by the storage of the isoxazole compound at room temperature under ordinary conditions was the same as the above-mentioned decomposition product (II), the stability of the isoxazole compound was thereafter investigated by measuring the amount of the decomposition product (II) produced.
The term "tight container" used in the present description and claims means the container defined in the 15th Edition of the Japanese Pharmacopoeia as follows: "A tight container protects the contents from extraneous solids or liquids, from loss of the contents, and from efflorescence, deliquescence, or evaporation under ordinary or customary conditions of handling, shipment, and storage." (corresponding to a tight container according to the foreign Pharmacopoeia). Specifically, the tight container refers to the glass bottle described in the present description as well as a plastic container of high-density polyethylene (HDPE) or the like, or a blister package of polyvinyl chloride (PVC), polypropylene (PP) or the like. A test using the glass bottle is preferable.

### Reference Example 2

### Preparation of a pulverized product of β type crystals of the isoxazole compound

With a pneumatic grinding mill Counter Jet Mill 100AFG (mfd. by Hosokawa Micron Corporation), β type crystals of the isoxazole compound were ground. The particle size of the pulverized product was measured with a laser diffraction type particle size distribution measuring apparatus HELOS & RODOS (mfd. by SYMPATEC) to find that the D50% particle size (a median diameter calculated on a volume basis) was 3.4 µm.
In the following working examples and comparative examples, there was used a pulverized product of β type crystals of the isoxazole compound having a D50% particle size of 2 to 7 µm and obtained by grinding in the same manner as above.

### Example 1

### Stabilization of the starting drug

250 Grams of the pulverized product described in Reference Example 2 was charged into a fluidized-bed granulator Multiplex MP-01 (mfd. by Powrex), sprayed with a solution of 2 mg of sodium metabisulfite in 300 g of purified water, and then dried.

### Example 2

### Stabilization of the starting drug

250 Grams of the pulverized product described in Reference Example 2 was charged into a fluidized-bed granulator Multiplex MP-01 (mfd. by Powrex), sprayed with a solution of 0.4 g of sodium metabisulfite in 300 g of purified water, and then dried.

### Comparative Example 1

250 Grams of the pulverized product described in Reference Example 2 was charged into a fluidized-bed granulator Multiplex MP-01 (mfd. by Powrex), sprayed with 300 g of purified water containing no antioxidant, and then dried.

### Test Example 1

### Stabilizing effect of sodium metabisulfite on the starting drug

### (1) Stability test

Each of the dried products of the starting drug obtained in Examples 1 and 2 and Comparative Example 1 was placed in a brown glass bottle and stored at 25°C in a desiccator subjected to moisture conditioning with a saturated aqueous lithium chloride solution (relative humidity in the desiccator: about 11%). The amount of the decomposition product (II), i.e., a compound produced by the decomposition of the isoxazole compound was measured at the start of the storage and 3 months after the start.

### (2) Results

Table 1 shows the results of the stability test on the isoxazole compound in each dried product in terms of the amount (%) of the decomposition product (II) produced. The amount (%) of the decomposition product (II) produced was measured by a HPLC method using an absorptiometer, and corrected by the use of an absorption coefficient, and then the relative amount of the decomposition product (II) to the amount of the isoxazole compound was expressed as % by weight.

**[Table 1]**

| Table 1: Stabilizing effect of sodium metabisulfite | | | | |
|---|---|---|---|---|
| | | Example 1 | Example 2 | Comparative Example 1 |
| Amount of sodium metabisulfite (% by weight based on the weight of the isoxazole compound) | | 0.0008 | 0.16 | 0 |
| Amount of decomposition product (II) (%) | At the start of storage | 0.004 | 0.004 | 0.005 |
| | After 3 months of storage | 0.004 | 0.004 | 0.088 |

As can be seen from the results shown in Table 1, the amount of the decomposition product (II) was increased during the storage in the case of the dried product of Comparative Example 1. On the other hand, the increase of the amount of the decomposition product (II) was completely suppressed from the start of the storage in the case of the dried products of Example 1 and 2.

### Example 3

### Tablets containing dibutylhydroxytoluene

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous hydroxypropylmethyl cellulose solution, and then with a solution of dibutylhydroxytoluene (BHT) and hydroxypropylmethyl cellulose in a water/ethanol mixture (1:1) to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained (Opadry (a registered trade name) 03B48970 was purchased from Colorcon Japan).

**[Table 2]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Dibutylhydroxytoluene | 0.05 |
| Magnesium stearate | 1 |
| Film-coating portion | |
| Opadry (registered trade name) 03B48970 | 3 |
| Carnauba wax | Trace amount |
| Total | 128.05 mg |

In the same manner as above, pharmaceutical compositions containing dibutylhydroxytoluene (BHT) in an amount of 0.5 mg or 0.01 mg per tablet were produced.

### Comparative Example 2

### Tablets containing no antioxidant

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous hydroxypropylmethyl cellulose solution, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 3]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Magnesium stearate | 1 |
| Film-coating portion | |
| Opadry (registered trade name) 03B48970 | 3 |
| Carnauba wax | Trace amount |
| Total | 128 mg |

### Test Example 2

### Stability of tablets containing dibutylhydroxytoluene and tablets containing no dibutylhydroxytoluene

### (1) Stability test

Each of the pharmaceutical compositions described in Example 3 and the pharmaceutical composition described in Comparative Example 2 was placed in a brown glass bottle, and the bottle was tightly stoppered and then stored at 80°C or 25°C. The amount of the decomposition product (II), i.e., a compound produced by the decomposition of the isoxazole compound was measured at the start of the storage; after 2 weeks of the storage at 80°C; and after 3 months of the storage at 25°C.

### (2) Results

Table 2 shows the results of the stability test on the isoxazole compound in each pharmaceutical composition in terms of the amount (%) of the decomposition product (II) produced.

**[Table 4]**

| Table 2: Stability of tablets containing dibutylhydroxytoluene (BHT) and tablets containing no dibutylhydroxytoluene | | | | | |
|---|---|---|---|---|---|
| | | Example 3 | | | Comparative Example 2 |
| BHT content of each tablet (mg) | | 0.5 | 0.05 | 0.01 | 0 |
| Weight of each tablet (mg) | | 128.5 | 128.05 | 128.01 | 128 |
| Amount of BHT (% by weight based on the weight of the isoxazole compound) | | 2.5 | 0.25 | 0.05 | 0 |
| Amount of decomposition product (II) (%) | At the start of storage | 0.016 | 0.012 | 0.011 | 0.014 |
| | 80°C for 2 weeks | 0.052 | 0.033 | 0.064 | 0.306 |
| | 25°C for 3 months | 0.033 | 0.018 | 0.049 | 0.119 |

As can be seen from the results shown in Table 2, the increase of the amount of the decomposition product (II) during the storage was suppressed in the pharmaceutical compositions containing dibutylhydroxytoluene in an amount of 0.5 mg to 0.01 mg per tablet (2.5% by weight to 0.05% by weight based on the weight of the isoxazole compound) as compared with the pharmaceutical composition of Comparative Example 2.

### Example 4

### Tablets containing sodium metabisulfite

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous solution of hydroxypropylmethyl cellulose and sodium metabisulfite, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 5]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Sodium metabisulfite | 0.05 |
| Magnesium stearate | 1 |
| Film-coating portion | |
| Opadry (registered trade name) 03B48970 | 3 |
| Carnauba wax | Trace amount |
| Total | 128.05 mg |

In the same manner as above, pharmaceutical compositions containing sodium metabisulfite in an amount of 0.5 mg or 0.01 mg per tablet were produced.

### Eample 5

### Tablets containing sodium metabisulfite

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous solution of hydroxypropylmethyl cellulose and sodium metabisulfite, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 6]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Sodium metabisulfite | 0.005 |
| Magnesium stearate | 1 |
| Film-coating portion | |
| Opadry (registered trade name) 03B48970 | 3 |
| Carnauba wax | Trace amount |
| Total | 128.005mg |

### Example 6

### Tablets containing sodium metabisulfite

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous solution of hydroxypropylmethyl cellulose and sodium metabisulfite, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 7]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Sodium metabisulfite | 0.001 |
| Magnesium stearate | 1 |
| Film-coating portion | |
| Opadry® (registered trade name) 03B48970 | 3 |
| Carnauba wax | Trace amount |
| Total | 128.001mg |

In the same manner as above, pharmaceutical compositions containing sodium metabisulfite in an amount of 0.00016 mg, 0.0001 mg or 0.00001 mg per tablet were produced.

### Example 7

### Tablets containing sodium metabisulfite

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous solution of hydroxypropylmethyl cellulose and sodium metabisulfite, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 8]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Sodium metabisulfite | 10 |
| Magnesium stearate | 1 |
| Film-coating portion | |
| Opadry® (registered trade name) 03B48970 | 3 |
| Carnauba wax | Trace amount |
| Total | 138mg |

In the same manner as above, a pharmaceutical composition containing sodium metabisulfite in an amount of 20 mg per tablet was produced.

### Test Example 3

### Stability of tablets containing sodium metabisulfite

### (1) Stability test

Each of the pharmaceutical compositions described in Examples 4 and 5 was placed in a brown glass bottle, and the bottle was tightly stoppered and then stored at 80°C or 25°C. Separately, each of the pharmaceutical compositions described in Examples 4 and 5 was placed in another brown glass bottle and stored at 40°C and 75%RH without stoppering the bottle. The amount of the decomposition product (II), i.e., a compound produced by the decomposition of the isoxazole compound was measured at the start of the storage; after 2 weeks of the storage at 80°C; after 3 months or 6 months of the storage at 40°C and 75%RH; and after 3 months of the storage at 25°C.
Each of the pharmaceutical compositions described in Examples 6 and Comparative Example 2 was placed in a brown glass bottle, and the bottle was tightly stoppered and then stored at 80°C or 40°C. The amount of the decomposition product (II), i.e., a compound produced by the decomposition of the isoxazole compound was measured at the start of the storage; after 2 weeks of the storage at 80°C; and after 2 weeks of the storage at 40°C.
Each of the pharmaceutical compositions described in Examples 7 was placed in a brown glass bottle, and the bottle was tightly stoppered and then stored at 80°C or 25°C. Separately, each of the pharmaceutical compositions described in Examples 7 was placed in another brown glass bottle and stored at 40°C and 75%RH without stoppering the bottle. The amount of the decomposition product (II), i.e., a compound produced by the decomposition of the isoxazole compound was measured at the start of the storage; after 2 weeks of the storage at 80°C; after 3 months of the storage at 40°C and 75%RH; and after 3 months of the storage at 25°C.

### (2) Results

Table 3, Table 4 and Table 5 show the results of the stability test on the isoxazole compound in each pharmaceutical composition in terms of the amount (%) of the decomposition product (II) produced.

**[Table 9]**

| Table 3: Stability of tablets containing sodium metabisulfite | | | | | |
|---|---|---|---|---|---|
| | | Example 4 | | | Example 5 |
| Sodium metabisulfite content of each tablet (mg) | | 0.5 | 0.05 | 0.01 | 0.005 |
| Weight of each tablet (mg) | | 128.5 | 128.05 | 128.01 | 128.005 |
| Amount of sodium metabisulfite (% by weight based on the weight of the isoxazole compound) | | 2.5 | 0.25 | 0.05 | 0.025 |
| Amount of decomposition product (II) (%) | At the start of storage | 0.012 | 0.009 | 0.008 | 0.007 |
| | 80°C for 2 weeks | 0.019 | 0.010 | 0.079 | 0.129 |
| | 40°C75%RH for 3 months | 0.011 | 0.009 | 0.010 | 0.011 |
| | 40°C75%RH for 6 months | 0.013 | 0.015 | 0.024 | 0.037 |
| | 25°C for 3 months | 0.016 | 0.010 | 0.012 | 0.010 |

**[Table 10]**

| Table 4: Stability of tablets containing sodium metabisulfite | | | | | | |
|---|---|---|---|---|---|---|
| | | Example 6 | | | | Comparative Example 2 |
| Sodium metabisulfite content of each tablet (mg) | | 0.001 | 0.00016 | 0.0001 | 0.00001 | 0 |
| Weight of each tablet (mg) | | 128.001 | 128.00016 | 128.0001 | 128.00001 | 128 |
| Amount of sodium metabisulfite (% by weight based on the weight of the isoxazole compound) | | 0.005 | 0.0008 | 0.0005 | 0.00005 | 0 |
| Amount of decomposition product (II) (%) | At the start of storage | 0.009 | 0.008 | 0.008 | 0.006 | 0.008 |
| | 80°C for 2 weeks | 0.183 | 0.218 | 0.184 | 0.162 | 0.214 |
| | 40°C for 2 weeks | 0.007 | 0.064 | 0.066 | 0.067 | 0.063 |

**[Table 11]**

| Table 5: Stability of tablets containing sodium metabisulfite | | | |
|---|---|---|---|
| | | Example 7 | |
| Sodium metabisulfite content of each tablet (mg) | | 10 | 20 |
| Weight of each tablet (mg) | | 138 | 148 |
| Amount of sodium metabisulfite (% by weight based on the weight of the isoxazole compound) | | 50 | 100 |
| Amount of decomposition product (II) (%) | At the start of storage | 0.010 | 0.010 |
| | 80°C for 2 weeks | 0.067 | 0.038 |
| | 40°C 75%RH for 3 months | 0.016 | 0.013 |
| | 25°C for 3 months | 0.005 | 0.006 |

As can be seen from the results shown in Table 3, Table 4 and Table 5, the amount of the decomposition product (II) was not substantially increased in the pharmaceutical compositions containing sodium metabisulfite in an amount of 20 mg to 0.005 mg per tablet (100% by weight to 0.025% by weight based on the weight of the isoxazole compound) when the compositions were stored at 25°C for 3 months. The pharmaceutical composition containing sodium metabisulfite in an amount of 0.001 mg per tablet (0.005% by weight based on the weight of the isoxazole compound) was stable to such an extent that the amount of the decomposition product (II) was not substantially increased in the composition when the composition was stored at 40°C for 2 weeks. The pharmaceutical compositions containing sodium metabisulfite in an amount of 20 mg to 0.005 mg per tablet (100% by weight to 0.025% by weight based on the weight of the isoxazole compound) were stable to such an extent that the amount of the decomposition product (II) was not substantially increased in the compositions even when the compositions were stored under severe conditions of 40°C and 75%RH for 3 months. The pharmaceutical compositions containing sodium metabisulfite in an amount of 0.5 mg to 0.01 mg per tablet (2.5% by weight to 0.05% by weight based on the weight of the isoxazole compound) were more stable to such an extent that the amount of the decomposition product (II) was not substantially increased in the compositions even when the compositions were stored under severer conditions of 40°C and 75%RH for 6 months.
In the case of the storage under still severer conditions of 80°C for 2 weeks, the increase of the amount of the decomposition product (II) was smaller in the pharmaceutical compositions containing sodium metabisulfite in an amount of 20 mg to 0.005 mg per tablet (100% by weight to 0.025% by weight based on the weight of the isoxazole compound) than in the pharmaceutical composition of Comparative Example 2. Thus, the stabilizing effect was exhibited. The pharmaceutical compositions containing sodium metabisulfite in an amount of 20 mg to 0.01 mg per tablet (100% by weight to 0.05% by weight based on the weight of the isoxazole compound) were more stable to such an extent that the amount of the decomposition product (II) was 0.10% or less when the compositions were stored at 80°C for 2 weeks. Furthermore, the pharmaceutical compositions containing sodium metabisulfite in an amount of 0.5 mg to 0.05 mg per tablet (2.5% by weight to 0.25% by weight based on the weight of the isoxazole compound) were very stable to such an extent that the amount of the decomposition product (II) was not substantially increased even when the compositions were stored under severer conditions of 80°C for 2 weeks.

### Example 8

### Tablets containing sodium bisulfite

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous solution of hydroxypropylmethyl cellulose and sodium bisulfite, to obtain a granulated product. After magnesium stearate was blended with the granulated product obtained, the resulting blend was compressed into core tablets.
These core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 12]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Sodium bisulfite | 0.05 |
| Magnesium stearate | 1 |
| Film-coating portion | |
| Opadry (registered trade name) 03B48970 | 3 |
| Carnauba wax | Trace amount |
| Total | 128.05 mg |

In the same manner as above, pharmaceutical compositions containing sodium bisulfite in an amount of 0.5 mg, 0.1 mg or 0.01 mg per tablet were produced.

### Example 9

### Tablets containing sodium bisulfite

According to the recipe shown below, mannitol, corn starch and croscarmellose sodium were charged into a fluidized-bed granulator Multiplex MP-01 (mfd. by Powrex) and sprayed with a binding liquid obtained by dispersing and suspending the isoxazole compound (a pulverized product of β type crystals) in an aqueous solution of hydroxypropylmethyl cellulose and sodium bisulfite, to obtain a granulated product. After magnesium stearate was blended with the granulated product obtained, the resulting blend was compressed into core tablets.
These core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 13]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Sodium bisulfite | 0.005 |
| Magnesium stearate | 1 |
| Film-coating portion | |
| Opadry (registered trade name) 03B48970 | 3 |
| Carnauba wax | Trace amount |
| Total | 128.005 mg |

### Test Example 4

### Stability of tablets containing sodium bisulfite

### (1) Stability test

Each of the pharmaceutical compositions described in Examples 8 and 9 was placed in a brown glass bottle, and the bottle was tightly stoppered and then stored at 80°C or 25°C. Separately, each of the pharmaceutical compositions described in Examples 8 and 9 was placed in another brown glass bottle and stored at 40°C and 75%RH without stoppering the bottle. The amount of the decomposition product (II), i.e., a compound produced by the decomposition of the isoxazole compound was measured at the start of the storage; after 2 weeks of the storage at 80°C; after 6 months of the storage at 40°C and 75%RH; and after 3 months of the storage at 25°C.

### (2) Results

Table 6 shows the results of the stability test on the isoxazole compound in each pharmaceutical composition in terms of the amount (%) of the decomposition product (II) produced.

**[Table 14]**

| Table 6: Stability of tablets containing sodium bisulfite | | | | | | |
|---|---|---|---|---|---|---|
| | | Example 8 | | | | Example 9 |
| Sodium bisulfite content of each tablet (mg) | | 0.5 | 0.1 | 0.05 | 0.01 | 0.005 |
| Weight of each tablet (mg) | | 128.5 | 128.1 | 128.05 | 128.01 | 128.005 |
| Amount of sodium bisulfite (% by weight based on the weight of the isoxazole compound) | | 2.5 | 0.5 | 0.25 | 0.05 | 0.025 |
| Amount of decomposition product (II) (%) | At the start of storage | 0.014 | 0.012 | 0.012 | 0.009 | 0.011 |
| | 80°C for 2 weeks | 0.011 | 0.012 | 0.014 | 0.204 | 0.113 |
| | 40°C75%RH for 6 months | No data | 0.013 | 0.014 | 0.049 | 0.031 |
| | 25°C for 3 months | 0.008 | 0.008 | 0.009 | 0.008 | 0.009 |

As can be seen from the results shown in Table 6, the amount of the decomposition product (II) was not substantially increased in the pharmaceutical compositions containing sodium bisulfite in an amount of 0.5 mg to 0.005 mg per tablet (2.5% by weight to 0.025% by weight based on the weight of the isoxazole compound) when the compositions were stored at 25°C for 3 months. The pharmaceutical compositions containing sodium bisulfite in an amount of 0.1 mg to 0.05 mg per tablet (0.5% by weight to 0.25% by weight based on the weight of the isoxazole compound) were more stable to such an extent that the amount of the decomposition product (II) was not substantially increased in the compositions even when the compositions were stored under severer conditions of 40°C and 75%RH for 6 months. The pharmaceutical compositions containing sodium bisulfite in an amount of 0.5 mg to 0.05 mg per tablet (2.5% by weight to 0.25% by weight based on the weight of the isoxazole compound) were more stable to such an extent that the amount of the decomposition product (II) was not substantially increased in the compositions even when the compositions were stored under severe conditions of 80°C for 2 weeks.

### Example 10

### Tablets containing sodium metabisulfite

According to the recipe shown below, the isoxazole compound (a pulverized product of β type crystals), mannitol, corn starch and croscarmellose sodium were charged into a fluidized-bed granulator Multiplex MP-01 (mfd. by Powrex) and sprayed with a binding liquid obtained by dissolving hydroxypropylmethyl cellulose and sodium metabisulfite in purified water, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 15]**

| Component | Content (mg) |
|---|---|
| Core tablet portion | |
| Isoxazole compound | 20 |
| Mannitol | 65.8 |
| Corn starch | 28 |
| Croscarmellose sodium | 6 |
| Hydroxypropylmethyl cellulose | 4 |
| Sodium metabisulfite | 0.05 |
| Magnesium stearate | 1 |
| Film-coating portion | |
| Opadry (registered trade name) 03B48970 | 3 |
| Carnauba wax | Trace amount |
| Total | 128.05 mg |

### Comparative Example 3

### Tablets containing no sodium metabisulfite

According to the recipe shown below, the isoxazole compound (a pulverized product of β type crystals), mannitol, corn starch and croscarmellose sodium were charged into a fluidized-bed granulator Multiplex MP-01 (mfd. by Powrex) and sprayed with a binding liquid obtained by dissolving hydroxypropylmethyl cellulose in purified water, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 3 mg of coating films, whereby film-coated tablets according to the recipe shown in the following table were obtained.

**[Table 16]**

| Component | Content (mg) |
|---|---|
| Core tablet portion | |
| Isoxazole compound | 20 |
| Mannitol | 66 |
| Corn starch | 28 |
| Croscarmellose sodium | 6 |
| Hydroxypropylmethyl cellulose | 4 |
| Magnesium stearate | 1 |
| Film-coating portion | |
| Opadry (registered trade name) 03B48970 | 3 |
| Carnauba wax | Trace amount |
| Total | 128 mg |

### Test Example 5

### Stability of tablets containing sodium metabisulfite and tablets containing no sodium metabisulfite

### (1) Stability test

Each of the pharmaceutical composition described in Example 10 and the pharmaceutical composition described in Comparative Example 3 was placed in a brown glass bottle, and the bottle was tightly stoppered and then stored at 80°C or 25°C. Separately, each of the pharmaceutical composition described in Examples 10 and the pharmaceutical composition described in Comparative Example 3 was placed in another brown glass bottle and stored at 40°C and 75%RH without stoppering the bottle. The amount of the decomposition product (II), i.e., a compound produced by the decomposition of the isoxazole compound was measured at the start of the storage; after 2 weeks of the storage at 80°C; after 6 months of the storage at 40°C and 75%RH; and after 3 months of the storage at 25°C.

### (2) Results

Table 7 shows the results of the stability test on the isoxazole compound in each pharmaceutical composition in terms of the amount (%) of the decomposition product (II) produced.

**[Table 17]**

| Table 7: Stability of tablets containing sodium metabisulfite and tablets containing no sodium metabisulfite | | | |
|---|---|---|---|
| | | Example 10 | Comparative Example 3 |
| Sodium metabisulfite content of each tablet (mg) | | 0.05 | 0 |
| Weight of each tablet (mg) | | 128.05 | 128 |
| Amount of sodium metabisulfite (% by weight based on the weight of the isoxazole compound) | | 0.25 | 0 |
| Amount of decomposition product (II) (%) | At the start of storage | 0.010 | 0.012 |
| | 80°C for 2 weeks | 0.013 | 0.173 |
| | 40°C75%RH for 6 months | 0.018 | 0.047 |
| | 25°C for 3 months | 0.011 | 0.116 |

As can be seen from the results shown in Table 7, the pharmaceutical composition described in Example 10 was so stable that the amount of the decomposition product (II) was not substantially increased under any storage conditions among the storage conditions described above.

### Example 11

### Tablets containing sodium metabisulfite

According to the recipe shown below, the isoxazole compound (a pulverized product of β type crystals), mannitol, corn starch and croscarmellose sodium were charged into a fluidized-bed granulator Multiplex MP-01 (mfd. by Powrex) and sprayed with a binding liquid obtained by dissolving hydroxypropylmethyl cellulose and sodium metabisulfite in purified water, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 5 mg of coating films, whereby film-coated tablets according to the recipe shown in the following table were obtained.

**[Table 18]**

| Component | Content (mg) |
|---|---|
| Core tablet portion | |
| Isoxazole compound | 40 |
| Mannitol | 131.6 |
| Corn starch | 56 |
| Croscarmellose sodium | 12 |
| Hydroxypropylmethyl cellulose | 8 |
| Sodium metabisulfite | 0.4 |
| Magnesium stearate | 1 |
| Film-coating portion | |
| Opadry (registered trade name) 03B48970 | 5 |
| Carnauba wax | Trace amount |
| Total | 255 mg |

In the same manner as above, pharmaceutical compositions containing sodium metabisulfite in an amount of 0.8 mg or 0.2 mg per tablet were produced.

### Comparative Example 4

### Tablets containing no sodium metabisulfite

According to the recipe shown below, the isoxazole compound (a pulverized product of β type crystals), mannitol, corn starch and croscarmellose sodium were charged into a fluidized-bed granulator Multiplex MP-01 (mfd. by Powrex) and sprayed with a binding liquid obtained by dissolving hydroxypropylmethyl cellulose in purified water, to obtain a granulated product. After magnesium stearate was blended with the granulated product obtained, the resulting blend was compressed into core tablets.
The core tablets were charged into Hi-Coater HCT-30N (mfd. by Freund Industrial Co., Ltd.) and coated so as to have 5 mg of coating films, whereby film-coated tablets according to the recipe shown in the following table were obtained.

**[Table 19]**

| Component | Content (mg) |
|---|---|
| Core tablet portion | |
| Isoxazole compound | 40 |
| Mannitol | 132 |
| Corn starch | 56 |
| Croscarmellose sodium | 12 |
| Hydroxypropylmethyl cellulose | 8 |
| Magnesium stearate | 1 |
| Film-coating portion | |
| Opadry (registered trade name) 03B48970 | 5 |
| Carnauba wax | Trace amount |
| Total | 255 mg |

### Test Example 6

### Stability of tablets containing sodium metabisulfite and tablets containing no sodium metabisulfite

### (1) Stability test

Each of the pharmaceutical compositions described in Example 11 and the pharmaceutical composition described in Comparative Example 4 was placed in a brown glass bottle, and the bottle was tightly stoppered and then stored at 40°C or 25°C. The amount of the decomposition product (II), i.e., a compound produced by the decomposition of the isoxazole compound was measured at the start of the storage; after 3 months of the storage at 40°C; and after 3 months of the storage at 25°C.

### (2) Results

Table 8 shows the results of the stability test on the isoxazole compound in each pharmaceutical composition in terms of the amount (%) of the decomposition product (II) produced.

**[Table 20]**

| Table 8: Stability of tablets containing sodium metabisulfite and tablets containing no sodium metabisulfite | | | | | |
|---|---|---|---|---|---|
| | | Example 11 | | | Comparative Example 4 |
| Sodium metabisulfite content of each tablet (mg) | | 0.8 | 0.4 | 0.2 | 0 |
| Weight of each tablet (mg) | | 255 | 255 | 255 | 255 |
| Amount of sodium metabisulfite (% by weight based on the weight of the isoxazole compound) | | 2 | 1 | 0.5 | 0 |
| Amount of decomposition product (II) (%) | At the start of storage | 0.02 | 0.02 | 0.02 | 0.02 |
| | 40°C for 3 months | 0.02 | 0.02 | 0.02 | 0.14 |
| | 25°C for 3 months | 0.02 | 0.02 | 0.02 | 0.19 |

As can be seen from the results shown in Table 8, the pharmaceutical compositions containing sodium metabisulfite in an amount of 0.8 mg, 0.4 mg or 0.2 mg per tablet (2% by weight, 1% by weight or 0.5% by weight based on the weight of the isoxazole compound) were so stable that the amount of the decomposition product (II) was not increased in the compositions by either the storage at 40°C for 3 months or the storage at 25°C for 3 months.

### Example 12

### Granules containing an antioxidant

According to the recipe shown below, the isoxazole compound (a pulverized product of β type crystals), mannitol, corn starch and croscarmellose sodium were mixed and then kneaded together by the use of a binding liquid obtained by dissolving hydroxypropylmethyl cellulose and sodium metabisulfite in purified water. The kneaded product was dried and then subjected to particle size regulation, and the granulated product thus obtained was incorporated with magnesium stearate to obtain granules.

**[Table 21]**

| Component | Content (mg) |
|---|---|
| Isoxazole compound | 5 |
| Mannitol | 77 |
| Corn starch | 32 |
| Croscarmellose sodium | 6 |
| Hydroxypropylmethyl cellulose | 4 |
| Sodium metabisulfite | 0.5 |
| Magnesium stearate | 1 |
| Total | 125.5 mg |

Granules were prepared in the same manner as above except for using each of sodium sulfite, erythorbic acid, sodium edetate and cysteine hydrochloride as an antioxidant in the same weight as above in place of sodium metabisulfite.

### Comparative Example 5

### Granules containing no antioxidant

According to the recipe shown below, the isoxazole compound (a pulverized product of β type crystals), mannitol, corn starch and croscarmellose sodium were mixed and then kneaded together by the use of a binding liquid obtained by dissolving hydroxypropylmethyl cellulose in purified water. The kneaded product was dried and then subjected to particle size regulation, and the granulated product thus obtained was incorporated with magnesium stearate to obtain granules.

**[Table 22]**

| Component | Content (mg) |
|---|---|
| Isoxazole compound | 5 |
| Mannitol | 77 |
| Corn starch | 32 |
| Croscarmellose sodium | 6 |
| Hydroxypropylmethyl cellulose | 4 |
| Magnesium stearate | 1 |
| Total | 125 mg |

### Comparative Example 6

### Granules containing sodium nitrite

According to the recipe shown below, the isoxazole compound (a pulverized product of β type crystals), mannitol, corn starch and croscarmellose sodium were mixed and then kneaded together by the use of a binding liquid obtained by dissolving hydroxypropylmethyl cellulose and sodium nitrite in purified water. The kneaded product was dried and then subjected to particle size regulation, and the granulated product thus obtained was incorporated with magnesium stearate to obtain granules.

**[Table 23]**

| Component | Content (mg) |
|---|---|
| Isoxazole compound | 5 |
| Mannitol | 77 |
| Corn starch | 32 |
| Croscarmellose sodium | 6 |
| Hydroxypropylmethyl cellulose | 4 |
| Sodium nitrite | 0.5 |
| Magnesium stearate | 1 |
| Total | 125.5 mg |

### Test Example 7

### Stability of granules containing an antioxidant and granules containing no antioxidant

### (1) Stability test

Each of the pharmaceutical compositions containing 0.5 mg of sodium metabisulfite, sodium sulfite, erythorbic acid, sodium edetate or cysteine hydrochloride as an antioxidant and produced by the process described in Example 12, the pharmaceutical composition containing no antioxidant of Comparative Example 5 and the pharmaceutical composition containing 0.5 mg of sodium sulfite of Comparative Example 6 was placed in a brown glass bottle, and the bottle was tightly stoppered and then stored at 80°C or 25°C. The amount of the decomposition product (II), i.e., a compound produced by the decomposition of the isoxazole compound was measured at the start of the storage; after 1 week of the storage at 80°C; and after 1 month of the storage at 25°C.

### (2) Results

Table 9 shows the results of the stability test on the isoxazole compound in each pharmaceutical composition in terms of the amount (%) of the decomposition product (II) produced.

**[Table 24]**

| Table 9: Stability of granules containing an antioxidant and granules containing no antioxidant | | | | | | | | |
|---|---|---|---|---|---|---|---|---|
| | | Example 12 | | | | | Comparative Example 5 | Comparative Example 6 |
| Antioxidant | | Sodium metabisulfite | Erythorbic acid | Sodium edetate | Cysteine hydrochloride | Sodium sulfite | Not added | Sodium nitrite |
| Content of antioxidant per 5 mg of the isoxazole compound (mg) | | 0.5 | 0.5 | 0.5 | 0.5 | 0.5 | 0 | 0.5 |
| Amount of antioxidant (% by weight based on the weight of the isoxazole compound) | | 10 | 10 | 10 | 10 | 10 | 0 | 10 |
| Amount of decomposition product (II) (%) | At the start of storage | 0.035 | 0.032 | 0.033 | 0.037 | 0.011 | 0.032 | 0.287 |
| | 80°C for 1 week | 0.037 | 0.126 | 0.179 | 0.048 | 0.012 | 0.054 | 1.325 |
| | 25°C for 1 month | 0.041 | 0.038 | 0.042 | 0.036 | 0.012 | 0.164 | 0.298 |

As can be seen from the results shown in Table 9, the amount of the decomposition product (II) was increased in the pharmaceutical composition of Comparative Example 5 during 1 month of the storage at 25°C. On the other hand, the increase of the amount of the decomposition product (II) was suppressed in the pharmaceutical compositions of Example 12 in the case of the measurement after 1 month of the storage at 25°C. In the pharmaceutical composition of Comparative Example 6 containing sodium nitrite, the amount of the decomposition product (II) was markedly increased during 1 week of the storage at 80°C. In the pharmaceutical compositions containing sodium edetate or erythorbic acid as an antioxidant, the amount of the decomposition product (II) after 1 week of the storage at 80°C, i.e., after the storage under severe conditions was large. The pharmaceutical composition containing cysteine hydrochloride was good in stability even when stored at 80°C for 1 week, but it emitted an irritating odor. Therefore, the pharmaceutical compositions containing sodium metabisulfite or sodium sulfite were considered more preferable.

### Example 13

### Granules containing an antioxidant

According to the recipe shown below, the isoxazole compound (a pulverized product of β type crystals), mannitol, corn starch and croscarmellose sodium were mixed and then kneaded together by the use of a binding liquid obtained by dissolving hydroxypropylmethyl cellulose and dibutylhydroxytoluene in a mixture of water and ethanol (1 : 1). The kneaded product was dried and then subjected to particle size regulation, and the granulated product thus obtained was incorporated with magnesium stearate to obtain granules.

**[Table 25]**

| Component | Content (mg) |
|---|---|
| Isoxazole compound | 5 |
| Mannitol | 77 |
| Corn starch | 32 |
| Croscarmellose sodium | 6 |
| Hydroxypropylmethyl cellulose | 4 |
| Dibutylhydroxytoluene | 0.5 |
| Magnesium stearate | 1 |
| Total | 125.5 mg |

Granules were prepared in the same manner as above except for using each of butylhydroxyanisole (BHA) and propyl gallate as an antioxidant in the same weight as above in place of dibutylhydroxytoluene (BHT).

### Comparative Example 7

### Granules containing no antioxidant

According to the recipe shown below, the isoxazole compound (a pulverized product of β type crystals), mannitol, corn starch and croscarmellose sodium were mixed and then kneaded together by the use of a binding liquid obtained by dissolving hydroxypropylmethyl cellulose in a mixture of water and ethanol (1 : 1). The kneaded product was dried and then subjected to particle size regulation, and the granulated product thus obtained was incorporated with magnesium stearate to obtain granules.

**[Table 26]**

| Component | Content (mg) |
|---|---|
| Isoxazole compound | 5 |
| Mannitol | 77 |
| Corn starch | 32 |
| Croscarmellose sodium | 6 |
| Hydroxypropylmethyl cellulose | 4 |
| Magnesium stearate | 1 |
| Total | 125 mg |

### Test Example 8

### Stability of granules containing an antioxidant and granules containing no antioxidant

Each of the pharmaceutical compositions containing 0.5 mg of dibutylhydroxytoluene (BHT), butylhydroxyanisole (BHA) or propyl gallate as an antioxidant and produced by the process described in Example 13 and the pharmaceutical composition of Comparative Example 7 was placed in a brown glass bottle, and the bottle was tightly stoppered and then stored at 80°C or 25°C. The amount of the decomposition product (II), i.e., a compound produced by the decomposition of the isoxazole compound was measured at the start of the storage; after 1 week of the storage at 80°C; and after 1 month of the storage at 25°C.

### (2) Results

Table 10 shows the results of the stability test on the isoxazole compound in each pharmaceutical composition in terms of the amount (%) of the decomposition product (II) produced.

**[Table 27]**

| Table 10: Stability of granules containing an antioxidant and granules containing no antioxidant | | | | | |
|---|---|---|---|---|---|
| | | Example 13 | | | Comparative Example 7 |
| Antioxidant | | Butylhydoxytoluene (BHT) | Butylhydroxyanisole (BHA) | Propyl gallate | Not added |
| Content of antioxidant per 5 mg of the isoxazole compound (mg) | | 0.5 | 0.5 | 0.5 | 0 |
| Amount of antioxidant (% by weight based on the weight of the isoxazole compound) | | 10 | 10 | 10 | 0 |
| Amount of decomposition product (II) (%) | At the start of storage | 0.031 | 0.033 | 0.034 | 0.050 |
| | 80°C for 1 week | 0.040 | 0.155 | 0.062 | 0.102 |
| | 25°C for 1 month | 0.045 | 0.028 | 0.038 | 0.144 |

As can be seen from the results shown in Table 10, the amount of the decomposition product (II) was increased in the pharmaceutical composition of Comparative Example 7 during 1 month of the storage at 25°C. On the other hand, the increase of the amount of the decomposition product (II) was suppressed in the pharmaceutical compositions containing BHT, BHA or propyl gallate, in the case of the measurement after 1 month of the storage at 25°C. Particularly in the pharmaceutical composition containing BHT, the increase of the amount of the decomposition product (II) was very sufficiently suppressed even in the case of the measurement after 1 week of the storage at 80°C, i.e., after the storage under severe conditions. Therefore, the pharmaceutical composition containing BHT was considered more preferable like the pharmaceutical composition containing sodium metabisulfite in Test Example 7.

### INDUSTRIAL APPLICABILITY

According to the present invention, when a specific antioxidant(s) excluding nitrites such as sodium nitrite and potassium nitrite is used in combination with the isoxazole compound useful as a therapeutic or prophylactic agent for autoimmune diseases, inflammatory diseases and the like, the production of a compound by the decomposition of the isoxazole compound is suppressed, so that long-term stabilization of the isoxazole compound is achieved and that the stabilization of the isoxazole compound is achieved even under severe conditions. Therefore, a pharmaceutical composition for oral administration containing a specific antioxidant(s) together with the isoxazole compound is an excellent composition containing the isoxazole compound in a stabilized state.

## Claims

1. A method for stabilizing an isoxazole compound represented by the following formula I: 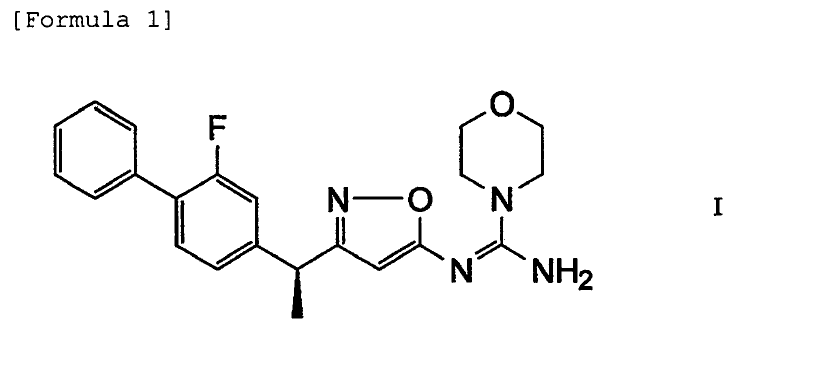 or a pharmaceutically acceptable salt thereof, **characterized by** stabilizing the isoxazole compound or the pharmaceutically acceptable salt thereof by suppressing the production of a compound of the following formula II: 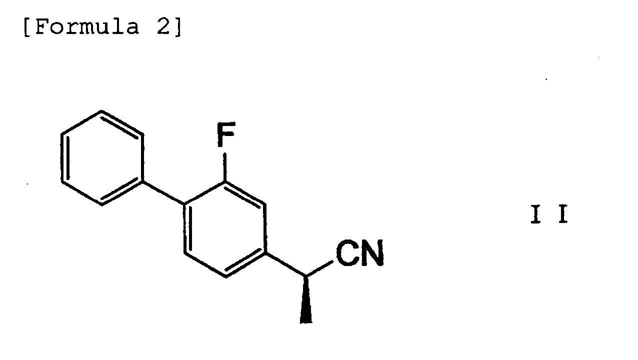 by the decomposition of the isoxazole compound or the pharmaceutically acceptable salt thereof, by the use of one or more antioxidants selected from metabisulfites; bisulfites; sulfites; erythorbic acid or salts thereof; edetic acid or salts thereof; cysteine hydrochloride; dibutylhydroxytoluene; butylhydroxyanisole; propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof.

2. The stabilization method according to claim 1, wherein the antioxidant(s) is at least one member selected from the group consisting of metabisulfites, bisulfites, sulfites, erythorbic acid or salts thereof, edetic acid or salts thereof, cysteine hydrochloride, dibutylhydroxytoluene, butylhydroxyanisole and propyl gallate.

3. The stabilization method according to claim 1 or 2, wherein the antioxidant(s) is at least one member selected from the group consisting of metabisulfites, bisulfites, sulfites and dibutylhydroxytoluene.

4. The stabilization method according to any one of claims 1 to 3, wherein the antioxidant(s) is at least one member selected from the group consisting of sodium metabisulfite, sodium bisulfite, sodium sulfite and dibutylhydroxytoluene.

5. The stabilization method according to any one of claims 1 to 4, wherein the antioxidant(s) is used in an amount of at least 0.0008% by weight based on the weight of the isoxazole compound or the pharmaceutically acceptable salt thereof.

6. The stabilization method according to any one of claims 1 to 5, wherein the antioxidant(s) is used in an amount of 100% by weight or less based on the weight of the isoxazole compound or the pharmaceutically acceptable salt thereof.

7. The stabilization method according to any one of claims 1 to 6, wherein the isoxazole compound of the above formula I is a pulverized product of β type crystals.

8. A pharmaceutical composition for oral administration comprising the isoxazole compound of the above formula I or a pharmaceutically acceptable salt thereof and one or more antioxidants selected from metabisulfites; bisulfites; sulfites; erythorbic acid or salts thereof; edetic acid or salts thereof; cysteine hydrochloride; dibutylhydroxytoluene; butylhydroxyanisole; propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof.

9. The pharmaceutical composition for oral administration according to claim 8, wherein the antioxidant(s) is that intended for suppressing the production of the compound of the above formula II by the decomposition.

10. The pharmaceutical composition for oral administration according to claim 8 or 9, wherein the antioxidant(s) is at least one member selected from the group consisting of metabisulfites, bisulfites, sulfites, erythorbic acid or salts thereof, edetic acid or salts thereof, cysteine hydrochloride, dibutylhydroxytoluene, butylhydroxyanisole and propyl gallate.

11. The pharmaceutical composition for oral administration according to any one of claims 8 to 10, wherein the antioxidant(s) is at least one member selected from the group consisting of metabisulfites, bisulfites, sulfites and dibutylhydroxytoluene.

12. The pharmaceutical composition for oral administration according to any one of claims 8 to 11, wherein the antioxidant(s) is at least one member selected from the group consisting of sodium metabisulfite, sodium bisulfite, sodium sulfite and dibutylhydroxytoluene.

13. The pharmaceutical composition for oral administration according to any one of claims 8 to 12, which comprises the antioxidant(s) in an amount of at least 0.0008% by weight based on the weight of the isoxazole compound or the pharmaceutically acceptable salt thereof.

14. The pharmaceutical composition for oral administration according to any one of claims 8 to 13, which comprises the antioxidant(s) in an amount of at least 0.005% by weight based on the weight of the isoxazole compound or the pharmaceutically acceptable salt thereof.

15. The pharmaceutical composition for oral administration according to any one of claims 8 to 14, which comprises the antioxidant(s) in an amount of at least 0.025% by weight based on the weight of the isoxazole compound or the pharmaceutically acceptable salt thereof.

16. The pharmaceutical composition for oral administration according to any one of claims 8 to 15, which comprises the antioxidant(s) in an amount of at least 0.05% by weight based on the weight of the isoxazole compound or the pharmaceutically acceptable salt thereof.

17. The pharmaceutical composition for oral administration according to any one of claims 8 to 16, which comprises the antioxidant(s) in an amount of at least 0.25% by weight based on the weight of the isoxazole compound or the pharmaceutically acceptable salt thereof.

18. The pharmaceutical composition for oral administration according to any one of claims 8 to 17, which comprises the antioxidant(s) in an amount of 100% by weight or less based on the weight of the isoxazole compound or the pharmaceutically acceptable salt thereof.

19. The pharmaceutical composition for oral administration according to any one of claims 8 to 18, which is in the form of tablets or granules.

20. The pharmaceutical composition for oral administration according to claim 19, wherein the tablets are film-coated tablets.

21. A pharmaceutical composition for oral administration comprising the following components (1) to (5) in the following proportions by weight:
(1) the isoxazole compound of the above formula I or a pharmaceutically acceptable salt thereof: 0.01% by weight to 25% by weight;
(2) a water-soluble excipient: 35% by weight to 90% by weight;
(3) a disintegrating agent: 1% by weight to 40% by weight;
(4) a water-soluble binder: 1% by weight to 10% by weight; and
(5) one or more antioxidants for suppressing the production of the compound of the above formula II by the decomposition which are selected from metabisulfites; bisulfites; sulfites; erythorbic acid or salts thereof; edetic acid or salts thereof; cysteine hydrochloride; dibutylhydroxytoluene; butylhydroxyanisole; propyl gallate; ascorbic acid or salts thereof; ascorbic acid esters; alpha-thioglycerol; citric acid or salts thereof; tocopherols; tocopherol esters; lecithin; thioglycolic acid or salts thereof; and thiomalic acid or salts thereof: at least 0.0008% by weight based on the weight of the isoxazole compound of the above formula I or the pharmaceutically acceptable salt thereof.

22. The pharmaceutical composition for oral administration according to claim 21, which comprises the antioxidant(s) in an amount of at least 0.005% by weight based on the weight of the isoxazole compound of the above formula I or the pharmaceutically acceptable salt thereof.

23. The pharmaceutical composition for oral administration according to claim 21 or 22, wherein the antioxidant(s) is at least one member selected from the group consisting of metabisulfites, bisulfites, sulfites, erythorbic acid or salts thereof, edetic acid or salts thereof, cysteine hydrochloride, dibutylhydroxytoluene, butylhydroxyanisole and propyl gallate.

24. The pharmaceutical composition for oral administration according to any one of claims 21 to 23, wherein the antioxidant(s) is at least one member selected from the group consisting of metabisulfites, bisulfites, sulfites and dibutylhydroxytoluene.

25. The pharmaceutical composition for oral administration according to any one of claims 21 to 24, wherein the antioxidant(s) is at least one member selected from the group consisting of sodium metabisulfite, sodium bisulfite, sodium sulfite and dibutylhydroxytoluene.

26. The pharmaceutical composition for oral administration according to any one of claims 21 to 25, wherein the content of the isoxazole compound or the pharmaceutically acceptable salt thereof is not more than 25% by weight and not less than 0.1% by weight.

27. The pharmaceutical composition for oral administration according to any one of claims 21 to 26, wherein the content of the isoxazole compound or the pharmaceutically acceptable salt thereof is not more than 25% by weight and not less than 1% by weight.

28. The pharmaceutical composition for oral administration according to any one of claims 21 to 27, wherein the content of the antioxidant(s) is not more than 100% by weight and not less than 0.025% by weight based on the weight of the isoxazole compound or the pharmaceutically acceptable salt thereof.

29. The pharmaceutical composition for oral administration according to any one of claims 21 to 28, wherein the content of the antioxidant(s) is not more than 100% by weight and not less than 0.05% by weight based on the weight of the isoxazole compound or the pharmaceutically acceptable salt thereof.

30. The pharmaceutical composition for oral administration according to any one of claims 21 to 29, wherein the content of the antioxidant(s) is not more than 10% by weight and not less than 0.25% by weight based on the weight of the isoxazole compound or the pharmaceutically acceptable salt thereof.

31. The pharmaceutical composition for oral administration according to any one of claims 21 to 30, wherein the content of the water-soluble binder is not more than 5% by weight and not less than 1% by weight.

32. The pharmaceutical composition for oral administration according to any one of claims 21 to 31, the dosage form of which is tablets or granules.

33. The pharmaceutical composition for oral administration according to any one of claims 21 to 32, wherein the isoxazole compound of the above formula I is a pulverized product of β type crystals.

34. The pharmaceutical composition for oral administration according to any one of claims 21 to 33, wherein the water-soluble excipient is at least one member selected from the group consisting of lactose, mannitol, erythritol and xylitol.

35. The pharmaceutical composition for oral administration according to any one of claims 21 to 34, wherein the disintegrating agent is at least one member selected from the group consisting of croscalmellose sodium, sodium starch glycolate, crospovidone, carmellose calcium, low-substituted hydroxypropyl cellulose, partly pregelatinized starch and natural starch.

36. The pharmaceutical composition for oral administration according to any one of claims 21 to 35, wherein the water-soluble binder is at least one member selected from the group consisting of hydroxypropylmethyl cellulose, hydroxypropyl cellulose, polyvinylpyrrolidones, polyvinyl alcohols and pullulan.

37. The pharmaceutical composition for oral administration according to any one of claims 21 to 36, which is in such a stabilized state that when the composition is stored in a tight container at 25°C for 3 months, the amount of the compound of the above formula II produced by the decomposition is about 0.1% by weight or less based on the weight of the isoxazole compound of the formula I.

38. A pharmaceutical composition for oral administration which comprises the following components
(1) to (5) in the following proportions by weight and is in such a stabilized state that when the composition is stored in a tight container at 25°C for 3 months, the amount of the compound of the above formula II produced by the decomposition is about 0.1% by weight or less based on the weight of the isoxazole compound of the formula I:
(1) the isoxazole compound of the above formula I or a pharmaceutically acceptable salt thereof: 0.01% by weight to 25% by weight;
(2) a water-soluble excipient: 35 to 90% by weight;
(3) a disintegrating agent: 1 to 40% by weight;
(4) a water-soluble binder: 1 to 10% by weight; and
(5) at least one antioxidant selected from the group consisting of sodium metabisulfite, sodium bisulfite, sodium sulfite and dibutylhydroxytoluene: not more than 100% by weight and not less than 0.005% by weight based on the weight of the isoxazole compound of the above formula I or the pharmaceutically acceptable salt thereof.

39. The pharmaceutical composition for oral administration according to claim 38, which comprises the antioxidant(s) in a proportion by weight of not more than 50% by weight and not less than 0.025% by weight based on the weight of the isoxazole compound of the above formula I or the pharmaceutically acceptable salt thereof.

40. A pharmaceutical composition comprising the isoxazole compound of the above formula I or a pharmaceutically acceptable salt thereof in an amount of 90% by weight or more and at least one antioxidant selected from the group consisting of metabisulfites, bisulfites, sulfites and dibutylhydroxytoluene in an amount of 0.0008% by weight or more based on the weight of the isoxazole compound of the above formula I or the pharmaceutically acceptable salt thereof.
